# EUROPEAN PATENT APPLICATION

(11) **EP 4 809 949 A1**
(43) Date of publication of application: **23.09.2026**
(21) Application number: 26166026.0
(22) Date of filing: 19.03.2026
(51) Int. Cl.: A61B 17/70, A61B 17/86

(54) **BONE FIXATION SCREW AND FIXATION KIT FOR SPINAL IMPLANTS**

(30) Priority: 21.03.2025 IT 202500005862
(71) Applicant: Mohar, Janez, 6000 Capodistria (SI)
(72) Inventor: Mohar, Janez, 6000 Capodistria (SI)
(74) Representative: Petraz, Gilberto Luigi

(57) **Abstract**

The bone fixation screw (10) for spinal implantation features an elongated body (11) that extends between two opposite ends: the first end (14) and the second end (16). The elongated body (11) is designed with a first threaded portion (13) at the first end (14), a second threaded portion (15) at the second end (16) and a nonthreaded intermediate portion (17) with constant diameter (D) between said threaded portions (13, 15). Additionally, there is a fixation kit (30) that includes multiple fixing screws (10) and several screw-to-rod connection elements (20, 60).

## Description

### FIELD OF APPLICATION

The present document refers to a bone fixation screw and to a fixation kit for surgical implantation, comprising the fixation screw and at least a screw-to-rod connection element for connecting the screw to a reinforcement rod. The fixation screw and screw-to-rod connection element are designed for use in spinal implants, particularly in thoracic spine implants. These implants are intended to provide a connection between vertebrae and reinforcement rods and restore the correct alignment of the vertebrae of a patient's spine by means of a temporary or permanent internal fixation construct.

### STATE OF THE ART

The spine consists of a series of vertebrae, intervertebral discs and posterior facet joints, with two adjacent vertebrae, wherein the intervertebral disc between them and the corresponding two facet joints with associated ligamentous tissue form a motion segment (i. e. smallest functional component of the spine). These motion segments can flex, extend, bend sideways, and rotate. Each element of the motion segment contributes to the mechanical stability of the spine. For example, the intervertebral discs that separate adjacent vertebrae provide rigidity, which helps limit relative movement of the vertebrae during flexion, extension, axial rotation, and lateral flexion.

Under ideal conditions, the vertebrae are aligned, and the spine maintains a straight alignment when viewed in the anteroposterior direction while exhibiting a defined curve when viewed from the side (cervical lordosis, thoracic kyphosis, lumbar lordosis).

When vertebrae become misaligned with respect to the ideal position due to injury or disease, it can result in severe pain and additional injuries to surrounding stabilizing structures. Misalignment that rotates and curves the vertebrae in the frontal plane is generally referred to as scoliosis. When vertebrae shift from their ideal curvature ranges in the lateral plane, the condition is referred to as hyper- / hypokyphosis or hyper- / hypolordosis.

To try to restore the correct alignment of the vertebrae of the spine, it is known to carry on surgical intervention that typically involves attaching reinforcement rods to the vertebrae with connecting bone anchors to stabilize them and prevent movements while applying a controlled straightening force to reduce deformity.

Several types of techniques using different bone anchor devices are known for connecting reinforcement rods to vertebrae.

One technique involves the use of pedicle screws, which are inserted and tightened in pairs on opposite sides of the vertebrae. Each screw has a connector on top in the shape of a tulip that attaches to a reinforcement rod. The pedicle screws must be inserted in a direction of the pedicle, not violating the spinal canal or intervertebral canal and potentially compromising the corresponding nervous structures, making proper positioning challenging. The surgeon must create a hole in the bone, identify the correct angle for insertion, and then insert the screw. This process requires significant time and expertise to avoid damaging the major vessels, visceral organs, spinal nerves, medulla spinalis or cauda equina.

Although enabling technologies for pedicle screw placement such as Intraoperative X-ray imaging, navigation systems, robotics, and the 3D-printed guides are known, these tools tend to have accuracy issues, are expensive, time consuming, their utilisation requires an acceptance of various amounts of electromagnetic radiation dosage for the patient and the surgical team and there is a substantial learning curve associated with each pedicle screw placement technique.

Another known technique provides using as bone anchors metallic hooks that attach directly to the vertebrae (i. e. transverse process hook, pedicular hook, laminar hook) or polyester bands or metallic wires that connect to the vertebrae by passing underneath the lamina. Although the fixation of such elements does not require additional enabling technologies, their vertebral fixation strength and realiability are lower compared to pedicle screws, making internal fixation structures less rigid than structures entirely made with pedicle screws.

Various bone screws with multiple threaded portions have been proposed in the prior art. US 2018/092751 A1 discloses a bone screw for spinal interbody fusion having a distal threaded portion and a proximal threaded portion, designed for compression or decompression between two adjacent vertebral bodies. EP 374427A1 discloses an intervertebral stabilisation screw with distal and proximal bone fixation threads intended for transpedicular insertion, passing through the disc space to screw into two adjacent vertebrae. US 5360448 A discloses bone screws with multiple threaded regions for securing prostheses. US 9636159B1 discloses a multi-thread iliac screw for embedding in the iliac bone with a saddle connector for spinal stabilization rods.

However, none of these prior art documents disclose a bone fixation screw specifically designed to be inserted through vertebral bodies such as spinous process and transverse process of a same vertebra, in particular a thoracic vertebra, thereby providing a four-cortical bone purchase while avoiding the spinal canal entirely. Furthermore, the prior art does not teach a bone fixation screw that functions as an artificial lamina to which a reinforcement rod can be connected via either a sublaminar band or a tulip-shaped anchoring device.

Therefore, there is a need to develop an improved bone fixation screw, a bone-to-rod connecting device to be used with the fixing screw and an associated fixation kit for use in the thoracic spine that overcomes at least one of the limitations of the current state-of-the-art technology.

In particular, one purpose of the present invention is to create a bone fixation screw and a fixation kit suitable for use in thoracic spine, that allow to expedite the securing of reinforcement rods while simultaneously reducing risks to patient safety.

Another purpose of the invention is to develop a fixation screw that can be used in thoracic implants, thereby decreasing the number of components that need to be fixed to bone structures.

Additionally, another purpose of the invention is to realize a fixation screw and a fixation kit that allow for a wide margin of tolerance within a range of anchoring positions, while still ensuring precise positioning and stable fixation of the reinforcement rod.

Another objective is to design a fixation screw compatible with various types of connecting devices.

Furthermore, the invention aims to create a fixation screw that is relatively easy for surgeons to use free-hand without the assistance of X-ray imaging, navigation systems, automated robots etc.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the invention or variants to the main inventive idea.

In accordance with the above purposes and to solve the identified technical issues in a novel and original way, thereby providing considerable advantages over prior art, a bone fixation screw for spinal thoracic implants according to the present invention comprises an oblong body, that is an elongated body extending in longitudinal direction and comprising externally a first threaded portion at one end and a second threaded portion at the opposite end, wherein the threaded pitch of the first threaded portion is equal to the pitch of the second threaded portion. This means that the at least the distance between the threads on each portion and eventually other characteristics of the threads are substantially the same.

The fixation screw is configured to be anchored on consecutive vertebral bodies, such as spinous processes and/or transverse processes.

The first and the second threaded portion are made on the same elongated body and therefore, when the fixation screw is rotated, both the threaded portions rotate in the same manner.

Between the first and second threaded portions, the elongated body comprise a non-threaded intermediate section with a substantially constant diameter along its entire length.

Preferably, the length of the non-threaded intermediate portion may be greater than the lengths of both the first and second threaded portions, and is preferably comprised between 2 and 3 times the length of the longer of the first and second threaded portions. The length of the intermediate portion is preferably comprised between 50% and 80% of the total length L, more preferably within 50%-70%.

According to one aspect of the invention, the elongated body has a tubular shape, and is internally provided with a channel that runs from one end to the other.

This channel allows for the insertion of a guide wire, which aids in aligning the first and second holes on two processes (spinous process and transverse process). The screw can then be advanced along the guide wire to ensure proper alignment in the desired direction.

Additionally, since both threaded portions have the same thread characteristics, it is possible to simultaneously screw both ends of the screw into their respective processes without causing stress or compression or distraction on the bone structure.

According to another aspect of the invention, the first threaded portion has a first maximum diameter that is equal to that of the elongated body, while the second threaded portion has a second maximum diameter that is greater than the diameter of the elongated body. Preferably, the second maximum diameter slightly exceeds the first maximum diameter.

This design allows the fixation screw to be initially inserted through a hole made in the thoracic vertebra's spinous process. The remaining section of the elongated body can then be advanced until the first end reaches the transverse process and screwed to secure it with the first threaded portion, while the second end is simultaneously screwed to the spinous process using the second threaded portion.

According to another aspect of the present invention, the first threaded portion has a greater longitudinal extension than the second threaded portion - for example approximately twice the extension of the first one. This design makes the fixation screw versatile, allowing its use in patients of varying sizes by adapting to a greater or shorter distance between the respective processes where it will be anchored.

The present invention also refers to a fixation kit that includes a screw-to-rod connection element configured to attach a reinforcement rod to a patient's spine. The fixation kit includes multiple bone fixation screws according to the invention and a plurality of screw-to-rod connection elements - preferably one for each fixation screw.

According to one aspect of the invention, the screw-to-rod connection element is chosen between an anchoring device or a sublaminar band.

In the case of a sublaminar band, the reinforcement rod may be fixed to the spine by passing a standard sublaminar band under the intermediate non-threaded part of the fixation screw and fixing the sublaminar band to the reinforcement rod in a standard fashion. In this way, the fixation screw acts as a lamina, with the advantage of not having to open the spinal canal and pass the standard sublaminar band under the lamina.

According to one aspect of the invention, the anchoring device is provided with a screw hole suitable for receiving the fixation screw and a rod seat for receiving a reinforcement rod. Optionally, the anchoring device comprises locking means suitable to cooperate with the rod seat for locking the reinforcement rod into it.

According to one aspect of the invention, the anchoring device includes a first coupling element in which the screw hole is provided, suitable for coupling with the fixation screw, a second coupling element provided with the rod seat and an intermediate junction element that connects the first and second coupling elements.

According to one aspect of the invention, the intermediate junction element comprises a threaded internal cavity configured to engage with a threaded protruding portion on the first coupling element.

According to one aspect of the invention, the intermediate junction element has an hourglass shape, comprising an enlarged lower section suitable to cooperate and securely connect with the first coupling element, a narrow middle section, and an enlarged upper section. The narrow middle section and the enlarged upper section cooperate with a housing seat of the second coupling element, thereby obtaining a coupling with at least one degree of freedom. The enlarged upper section may have a rounded outer shape.

According to one aspect of the invention, the design of the junction element and the second coupling element permits the latter to tilt at a predefined angle relative to the vertical, for example, between 0° and 60° preferably at least between 0° and 45°, similar to the pedicle screws.

This configuration enables to connect the second coupling element with a degree of movement that facilitates the insertion and positioning of the reinforcement rod.

According to another aspect of the invention, the threaded protruding portion of the first coupling element comprises a cut along a vertical median plane that extends to the screw hole. This cut divides the threaded protruding portion into two halves that can move apart slightly within a certain range - preferably between 0.1° and 2° - to help with the insertion of the fixation screw's elongated body.

Once the fixation screw is inserted, the two halves can be drawn closer together and locked in position by means of the intermediate junction element, which ensures a stable connection between the fixation screw and the anchoring device, given the substantially equal size of the diameters of the screw hole and the intermediate portion of the fixation screw.

### ILLUSTRATION OF THE DRAWINGS

These and other aspects, features, and advantages of the invention will become apparent from the following description of some embodiments, provided by way of example and without limitation, with reference to the attached drawings, in which:
- Fig. 1 is a side view of a bone fixation screw according to the invention;
- Fig. 2 is an exploded three-dimensional view of a bone fixation screw and an anchoring device from a fixation kit according to the invention;
- Fig. 3 shows the components of Fig. 2 in an assembled condition in a first operating position;
- Fig. 4 shows the components from Fig. 2 in an assembled condition in a second operating position;
- Fig. 5 is a cross-sectional view of the intermediate junction element of the anchoring device according to the invention;
- Fig. 6 is a side view of the first coupling element of the anchoring device according to the invention;
- Fig. 7 is a schematic view of a portion of a spine with components of a fixation kit connected to three vertebrae.

It is important to note that the terminology used in this description, as well as the figures in the attached drawings, serve solely to improve the understanding of the invention and should not be considered as limiting. The scope of protection is defined by the claims.

To facilitate comprehension, identical reference numbers have been used, where possible, to denote common elements across the figures. It should be understood that the features and characteristics of one embodiment can be combined or integrated into other embodiments without further clarification.

### DESCRIPTION OF SOME EMBODIMENTS OF THE PRESENT INVENTION

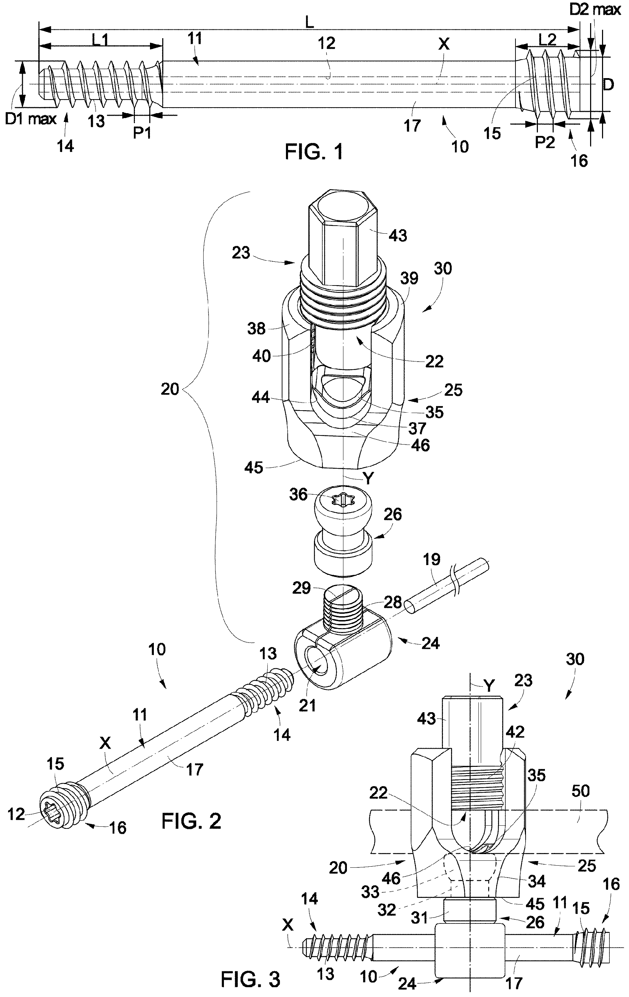

With reference to Figure 1, a bone fixation screw 10 according to the present invention, suitable for spinal thoracic implantation, includes an oblong, that is an elongated body 11 that extends longitudinally along an X-axis. The elongated body 11 may have a tubular shape and comprise an internal channel 12 that runs from one end to the other.

In channel 12, a guide wire 19 made of metal or other suitable material can be inserted to guide the connection of the fixation screw 10 to the bone structure as will be described below.

The fixation screw 10 comprises two threaded sections made externally on the elongated body 11: a first threaded portion 13 at one first end 14, referred to as the distal end, and a second threaded portion 15 at the opposite second end 16, referred to as proximal end. These threaded portions 13, 15 extend substantially to the ends of the oblong body 11. The fixation screw 10 is a headless screw, that is, it does not comprise any enlarged portion at either end 14, 16.

The pitch P1 and possibly other characteristics of the threads on the first portion 13 is/are equal to the pitch P2 and possibly other characteristics of the threads on the second portion 15.

The pitches P1 and P2 are equal to each other and typically ranging from 0.5mm to 2mm, preferably about 1mm.

Between the first 13 and second threaded portions 15, the elongated body 11 includes an intermediate not-threaded portion 17 with a substantially constant diameter D along its length.

The fixation screw 10 may be of the self-tapping or non-self-tapping type.

The first threaded portion 13 may have a first maximum diameter D1max that does not exceed the diameter D of the oblong body 11, that is that of the intermediate portion 17.

The second threaded portion 15 may have a second maximum diameter D2max that is larger than the diameter D of the oblong body 11, for example between 0.4mm to 1mm larger, preferably around 0.5 mm to 0.6 mm larger.

As depicted in the embodiment of Figure 1, the first threaded portion 13 extends longitudinally to a first length L1 that is greater than the second threaded portion 15, which has a second length L2. This design allows the fixation screw 10 to properly secure onto vertebrae with varying sizes and distances between the processes within a certain range.

According to some embodiments, the first length L1 can be about twice that of the second length L2 or generally between 1.1 and 2.5 times the second length L2.

Alternatively, other embodiments may provide the two lengths L1, L2 being approximately equal, or the second length L2 may exceed the first length L1-for example, ranging from 1.1 to 2.5 times L1. The fixation screw may have a ratio between diameter D and overall length L comprises between 0.05 and 0.15.

The fixation screw 10 can have a diameter D between 3.0mm and 5.0mm, with common choices being 3.5mm, 4.0mm, or 4.5mm, as well as various intermediate values, and an overall length L from 25mm to 60mm, such as 30mm, 35mm, 40mm, 45mm, 50mm, and 55mm, including intermediate values.

The lengths L1 and/or L2 may be comprised between 10% to 30% of the total length L.

Preferably, the length of the intermediate portion 17 may be greater than the lengths of both the first and second threaded portions 13, 15, and is preferably comprised between 2 and 3 times the length of both or the longer of the first and second threaded portions 13, 15.

The length of the intermediate portion 17 mat be comprised between 50% and 80% of the total length L, preferably within 50%-70%.

According to another aspect of the invention, the channel 12, or at least a portion of such channel 12, is shaped so as to accommodate the insertion and engagement of a tool, specifically a screwdriver, that has a compatible shape (not illustrated), such as a star shape with six points. For example, Figure 2 illustrates a shaped recess compatible with a T8 Torx screwdriver, although other shapes can also be utilized.

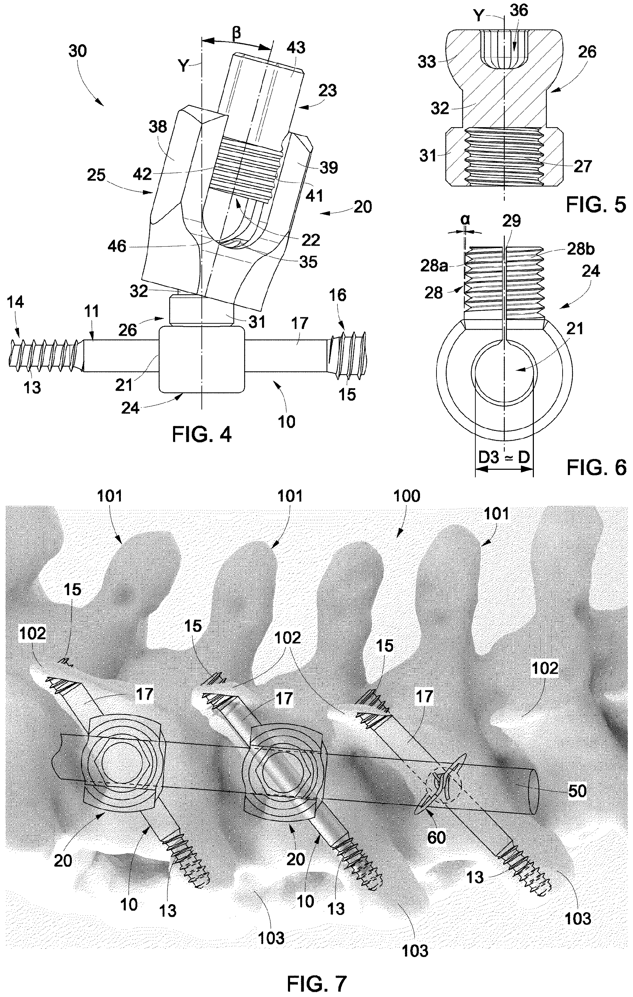

Figure 2 also shows components of a fixation kit 30 designed to secure a reinforcement rod 50, as illustrated by the dotted line in Figure 3, to the spine 100 of a patient (Figure 7).

The fixation kit 30 comprises a plurality of fixation screws 10 and a plurality of screw-to-rod connection elements 20, 60, preferably one for each fixation screw 10. The screw-to-rod connection elements 20, 60 can be selected between anchoring devices 20 (Figures 2-6) or sublaminar bands 60 (Figure 7).

The sublaminar bands 60 may be of a standard type, made in polyester or other suitable polymeric material. Such sublaminar bands 60 instead of being attached to the lamina of the spine 100 can be attached directly to the fixation screws 10.

Each anchoring device 20 has a screw hole 21 designed to accommodate the fixation screw 10 during use, and a rod seat 22 for accommodating the reinforcement rod 50 during use. The screw hole 21 has a diameter D3 that is approximately equal to the diameter D of the elongated body 11, preventing any unintended rotation of the fixation screw 10 during use.

The rod seat 22 is preferably "U"-shaped, open laterally and from the top, in order to allow the insertion of the reinforcement rod 50 axially into multiple anchoring devices 20.

Preferably, the anchoring device 20 includes locking means 23, preferably in form of set screws, configured to cooperate in conjunction with the rod seat 22 to secure the reinforcement rod 50 in place.

The anchoring device 20 may comprise a first coupling member 24, in which the screw hole 21 is made, a second coupling member 25, in which the rod seat 22 is made, and an intermediate junction member 26 configured to connect the first 24 and second 25 coupling members one to the other.

According to one aspect of the invention, the junction member 26 has a threaded internal cavity 27 suitable to receive and engage with a threaded protruding portion 28 provided on the first coupling member 24.

According to another aspect, the threaded protruding portion 28 is provided with a cut 29, that is a removed segment that creates an interstice, that is a gap extending along a vertical median plane up to the screw hole 21.

This interstice divides the threaded protruding portion 28 into two halves 28a and 28b, that mutually exhibit an albeit limited degree of freedom. The interstice defined by cut 29 can be between 1mm and 2mm wide, e.g. 1.5 mm or other intermediate values.

For example, the two halves 28a, 28b can be slightly spread/moved by a certain clearance angle α with respect to a vertical plane, e.g. between 0.1°-2° so as to slightly widen the screw hole 21 and facilitate the insertion of the fixation screw 10 into it.

When the fixation screw 10 is inserted, preferably with the intermediate portion 17 arranged in the screw hole 21, the two halves 28a, 28b can be brought closer together and locked in place by screwing the junction member 26 onto them.

According to one aspect of the invention, the junction member 26 has an hourglass conformation.

Referring to Figures 2 and 5, the junction member 26 includes an enlarged lower portion 31 in which the threaded internal cavity 27 is provided, a narrow intermediate portion 32, and an enlarged upper portion 33.

The enlarged portions 31, 33 may have substantially corresponding height and cross-sectional dimensions in a horizontal plane.

On the top of the upper portion 33 may be provided a shaped engagement seat 34 for inserting a tool, such as a T8 Torx 6-points screwdriver.

The conformation of the narrow intermediate portion 32 and the enlarged upper portion 33 allows a connection with a certain degree of freedom between junction member 26 and second coupling member 25.

In particular, the second coupling member 25 can tilt by an angle of inclination β with respect to a vertical axis Y that can be, for example, between 0°-60°, preferably between 0°-45°.

The enlarged upper portion 33 preferably has a rounded shape that allows it to rotate within a housing seat 34 provided in the upper second coupling member 25.

The upper second coupling member 25 can be inserted from above onto the junction member 26, and then an internal retaining ring 35 can be inserted into the housing seat 34 to lock the upper second coupling member 25 in the desired position.

In this regard, the housing seat 34 may be circumferentially closed and have respective upper 44 and lower passage openings 45.

The inner retaining ring 35, on the surface facing the rod seat 22, may have a recess 37 of concave shape suitable for supporting a reinforcement bar 50 of rounded section resting on it.

In possible variations of embodiments, not illustrated, it may be provided that the upper second coupling member 25 is stably connected to the junction member 26 by shape or screw coupling, although this solution does not allow the same versatility of positioning.

According to embodiments, the upper second coupling member 25 may comprise two opposite side walls 38, 39 delimiting the rod seat 22, and in particular defining the arms of the "U" shape; the bottom of the "U" being defined by a joining wall 46 between the two side walls 38, 39 and/or by an upper surface of the internal retaining ring 35 or an upper part of the housing seat 34.

The side walls 38 and 39 can be internally equipped with respective threaded portions 40 and 41, which are aligned in operational continuity with each other. These portions are designed to screw onto a common threaded section 42 of the locking means, 23, in particular made in form of set screws.

In this instance, the screw locking means 23 can take the form of a pin, comprising a threaded portion 42 at the bottom and a shaped grip portion 43 at the top, allowing for the insertion therein of a tool to facilitate its rotation.

According to embodiments, each anchoring device 20 consists of four main elements: a first coupling member 24, a second coupling member 25, a junction member 26, and the screw locking means 23, preferably made as set screws.

In alternative configurations, each anchoring device 20 may consist of five components: the first coupling member 24, the second coupling member25, the junction member 26, the screw locking means 23, preferably in form of set screws, and the internal retaining ring 35.

The fixation kit 30 may include not only the fixation screws 10 and anchoring devices 20 but also the corresponding locking means 23, preferably in the form of set screws, and one or more guide wires 19.

According to variant embodiments, the fixation kit 30 can also contain a plurality of sublaminar bands 60 and/or tie rods, designed to be attached to the fixation screws 10, in addition to or as an alternative to the anchoring devices 20.

In further implementations, the fixation kit 30 might incorporate one or more reinforcement rods 50 or be compatible with standard-sized reinforcement rods suitable for insertion into the rod seats 22.

For example, a standard reinforcement rod 50 usually has a diameter comprised between 5.5 mm and 6.0 mm, but other sizes can be provided, as long as the rod seat 22 and the reinforcement rod 50 have substantially conjugate shapes and sizes.

The following with reference to figure 7 provides a brief overview of a procedure, not claimed and not forming part of the present invention, for implanting a fixation screw 10 and an anchoring device 20 according to the invention.

The procedure begins with locating the entry points on the transverse process 103 and the spinous process 102. The entry point on the transverse process 103 is decorticated with the use of a high-speed burr and the entry point on the spinous process 102 is drilled through. Next, a guide wire 19 is inserted through a hole in the spinous process 102 and advanced in the direction to the decorticated pit on the transverse process 103. The guide wire 19 is pushed through the decorticated pit to touch the anterior cortex of the transverse process 103. A direct measuring device is slid over the free end of the guide wire 19 to gauge the appropriate fixation screw length.

The fixation screw hole is made with a cannulated drill bit over the guide wire 19 by drilling the definite hole through the spinous 102 and transverse process 103, respectively (all four cortices). The definite hole diameter corresponds to the diameter D of the elongated body 11, that is the intermediate portion 17 of the fixation screw 10. In case a non-self-tapping fixation screw 10 is used, the hole in the spinous and transverse process is tapped. This step can be skipped in case a self-tapping fixation screw 10 is used.

The method then involves inserting a guide wire 19 between two holes made on two articular processes of a vertebra 101, in the example case between a spinous process 102 and a transverse process 103 (similarly, the screws could be fixed between a transverse process 103 and the spinous process 102).

Next, the fixation screw 10 is inserted into the hole on the spinous process 102 with its first threaded portion 13.

If an anchoring device 20 is to be used, it is attached to the fixation screw 10 through the screw hole 21.

When the first end 14 of the fixation screw 10 is positioned between the spinous process 102 and the transverse process 103, it can then be inserted into the screw hole 21 within the first coupling member 24 of the anchoring device 20.

The fixation screw 10 is advanced until the first end 14, equipped with the first threaded portion 13, reaches the transverse process 103, while the second threaded portion 15 reaches the hole on the spinous process 102.

From this point, the fixation screw 10 is simultaneously tightened with the first threaded portion 13 onto the transverse process 103 and the second threaded portion 15 onto the spinous process 102 until a stable fixation is achieved between the bone and the fixation screw 10. Since the pitches P1, P2 on the first and second threaded portions 13, 15 are the same, the fixation screw 10 can be screwed into both processes 102, 103 without generating any tension in the bone portions.

After securing the fixation screw 10 to the bone structures, the junction member 26 and the second coupling member 25 can be attached to the first coupling member 24.

Once the respective fixation screws 10 and anchoring devices 20 are fixed to the pertinent vertebrae 101, the reinforcement rod 50 can be inserted into the corresponding rod seats 22, adjusting the inclination and positioning of the anchoring devices 20 as necessary.

Finally, the respective set screw locking means 23 can be fastened to each rod seat 22 to permanently secure the reinforcement rod 50 in the desired position, preventing any unintended movement.

Even if in real surgical implants are generally used screw-to-rod connection elements 20, 60 of the same type, figure 7 shows by way of illustration also a sublaminar band 60 connected to a fixation screw 10 and the reinforcement rod 50.

It is evident that modifications and/or additions to the fixation screw 10 and the fixation kit 30 can be made without departing from the overarching scope defined by the claims.

Additionally, while the current description refers to specific examples, a skilled practitioner will be able to create other equivalent versions of the fixation screws and fixation kits that exhibit the characteristics outlined in the claims, thereby falling within the defined scope of protection. It should be noted that references in parentheses within the claims are intended solely for clarity and should not be interpreted as limiting factors to the scope of protection.

## Claims

1. A bone fixation screw (10) for spinal implantation configured to be anchored in vertebral bodies (101, 103) of a vertebra (101), comprising an oblong body (11) that extends between opposite first and second ends (14, 16) that are respectively externally equipped with a first threaded portion (13) on the first end (14) and a second threaded portion (15) on the second end (16), wherein between said first and second threaded portions (13, 15) the elongated body (11) comprises a non-threaded intermediate portion (17) having a substantially constant diameter (D), **characterized in that** at least a threaded pitch (P1) of said first threaded portion (13) is equal to the thread pitch (P2) of said second threaded portion (15) and wherein said first threaded portion (13) has a maximum diameter (D1max) that is less than or equal to the diameter (D) of said intermediate portion (17) and said second threaded portion (15) has a maximum diameter (D2max) that is greater than said diameter (D).

2. The bone fixation screw (10) as in claim 1, **characterized in that** said first threaded portion (13) is configured to engage a transverse process (103) and said second threaded portion (15) is configured to engage a spinous process (102) of a same thoracic vertebra, thereby providing a four-cortical bone purchase.

3. The bone fixation screw (10) as in claim 1 or 2, **characterized in that** said elongated body (11) internally comprises a channel (12) that extends in longitudinal direction (X) between the first and second ends (14, 16).

4. The bone fixation screw (10) as in any of claims 1 to 3, **characterized in that** said first threaded portion (13) has a greater extension in longitudinal direction (X) than the second threaded portion (15), preferably between 1.2 and 2.5 times.

5. The bone fixation screw (10) as in any of claims 1 to 4, **characterized in that** a length of said non-threaded intermediate portion (17) is greater than the lengths of both the first and second threaded portions (13, 15), and is preferably comprised between 50-80% of a length of said elongated body (11).

6. The bone fixation screw (10) as in any of claims 1 to 5, **characterized in that** said bone fixation screw (10) is configured to function as an artificial lamina to which a reinforcement rod (50) can be connected via a sublaminar band (60) passed under said intermediate non-threaded portion (17) or by means of an anchoring device (20).

7. The bone fixation screw (10) as in any of claims 1 to 6, **characterized in that** the fixation screw (10) is a headless screw and said threaded portions (13, 15) extend substantially up to the respective ends (14, 16) of the elongated body (11).

8. A fixation kit (30) for spinal implantation, comprising a plurality of bone fixation screws (10) as in any of claims 1 to 7 and a plurality of screw-to-rod connection elements (20, 60) selected between anchoring devices (20) and/or sublaminar bands (60).

9. The fixation kit (30) of claim 8, **characterized in that** each anchoring device (20) is provided with a screw hole (21), where one of the fixation screws (10) can be placed during use, and a rod seat (22) where a reinforcement rod (50) can be fixed during use and optionally with locking means (23), preferably in the form of set screws, suitable to cooperate with the rod seat (22) to secure the reinforcement rod (50) therein.

10. The fixation kit (30) as in claim 9, **characterized in that** each anchoring device (20) includes a first coupling member (24) provided with the screw hole (21) and a second coupling member (25) provided with the rod seat (22), and an intermediate junction member (26) configured to connect said first and second coupling members (24, 25) with each other.

11. The fixation kit (30), as in claim 10, **characterized in that** said junction member (26) includes a threaded internal cavity (27) suitable for engagement with a threaded protruding portion (28) of said first coupling member (24).

12. The fixing kit (30) as described in claim 10 or 11, **characterized in that** said junction member (26) has a hourglass shape, comprising an enlarged lower portion (31) that cooperates and stably connects with the first coupling member (24), a narrow intermediate portion (32), and an enlarged upper portion (33), said narrow intermediate portion (32) and enlarged upper portion (33) being configured to cooperate with a housing seat (34) of said second coupling member (25), allowing for at least one degree of freedom in the coupling.

13. The fixation kit (30) as in any claims 8 to 12, **characterized in that** said second coupling member (25) is tiltable relative to said junction member (26) within an angle of inclination (β) ranging from 0° to 60°.

14. The fixation kit (30) as in any claim 8 to 13, **characterized in that** the first coupling member (24) includes a threaded protruding portion (28) with a cut (29) along a vertical median plane that extends to the screw hole (21), dividing the threaded protruding portion (28) into two movable halves (28a, 28b), each movable with a clearance angle (α) between 0.1° and 2°.

15. The fixation kit (30) as in any of claims 8 to 14, **characterized in that** said upper coupling member (25) features two opposite side walls (38, 39) that laterally define said rod seat (22), wherein each of the side walls (38, 39) is internally equipped with respective threaded portions (40, 41) that are in operational continuity with each other and suitable for threading onto a common threaded portion (42) of the locking means (23).
